Europäisches Patentamt

European Patent Office

Office européen des brevets

(19)

(11) Publication number : **0 472 282 A2**

## (12) EUROPEAN PATENT APPLICATION

(21) Application number : **91306438.2**

(22) Date of filing : **16.07.91**

(51) Int. Cl.⁵ : **G01N 3/20**

(30) Priority : **20.07.90 AU 1302/90**

(43) Date of publication of application :
**26.02.92 Bulletin 92/09**

(84) Designated Contracting States :
**AT BE CH DE DK ES FR GB GR IT LI LU NL SE**

(71) Applicant : **CSR LIMITED**
**Level 35, Grosvenor Place, 225 George Street**
**Sydney, New South Wales, 2000 (AU)**

(72) Inventor : **Tisch, Thomas**
**317 Bilsen Road Geebung**
**Queensland, 4034 (AU)**

(74) Representative : **Parry, Christopher Stephen et al**
**PAGE, WHITE & FARRER 54 Doughty Street**
**London WC1N 2LS (GB)**

(54) **Method and apparatus for continuous panel testing.**

(57)    An apparatus for the continuous testing of planar panels of particulate or layered material comprises a support structure (6) having an array of rollers (2,3,4,5,7,8) configured to facilitate continuous feeding and sandwiching of a panel (12) therebetween, and means (11) for applying a load to the panel to induce a deflection in the panel, and a load cell (13) which in conjunction with the roller (11) measures the deflection load, enabling the stiffness and related parameters of the panel to be calculated.

EP 0 472 282 A2

FIGURE 1

The present invention relates to a method and apparatus for continuous testing of panels such as particle board, plywood, fibreboard and light panels to determine the stiffness of a panel as it travels through the apparatus.

More particularly the invention is directed towards the non destructive testing of the stiffness of a panel utilising a novel configuration of deflection and support rollers which enable measurement of the stiffness of a panel by load application thereto. In industry and in particular, in the particle board and panel manufacturing industry, it is necessary to test the structural integrity of the panel to enable an assessment to be made of its structural characteristics and in order to grade the panels. One viable indicator of strenght of panels is the stiffness factor and this has been used in the past as a barometer of the structural integrity of a panel.

One method which has previously been used to determine the strength of a composite wood panel is to randomly or according to a predetermined plan select representative panels from a production population and by applying a load physically test them to destruction. From the result of these test, the stiffness and strength of the whole population produced is estimated. This method is inherently unsatisfactory on a number of grounds. Firstly, the test sample is physically destroyed to achieve the results; secondly, there is no guarantee that the result achieved with on panel can be accurately extrapolated over a whole population of panels and thirdly, it is a random selection from a population, thus introducing errors in the assessment of the strength of individual panels of sheets.

According to the prior art, there are also methods known to test sheets non destructively but these particular test are time consuming, inaccurate or difficult to perform. They are carried out on samples or randomly selected sheets only as in the destructive testing program previously mentioned. The non destructive testing methods of the prior art involve the determination of deflection in bending of sheets such as structural plywood, where the sheet to be tested is places in a jig and subjected to bending stress. The bending force and deflection are measured and a stiffness property is calculated. This method has the disadvantage that sheet has to be placed into the jig and removed again, which is a time consuming procedure. Furthermore, the sheet is generally tested in only one localised position on the sheet.

Another method of non destructive testing involves stress wave analysis. The testing of sheet under this regime is based on the relationship of the resonant frequency of the simply supported specimen and the modulus of elasticity of that specimen of the relationship of the rate of propogation of a sound wave in a specimen to the modulus of elasticity of that specimen. The disadvantage of this method result from the special handling of a sheet which is required for testing.

The present invention seeks to ameliorate the aforesaid problems of the prior art by providing a method and apparatus therefor for continuous and accurate testing of the structural characteristics, integrity and properties of panels to enable classification of the panels into structural classes.

According to the preferred embodiment, the testing would be performed on panels having a width to thickness ratio greater than 15 however, this ratio is not to be construed as limiting to the overall methodology.

The method and apparatus of the present invention allows for every sheet in a production run to be tested without additional handling and in a continuous process. Since each sheet is tested, the property of each sheet is known and therefore it can be identified which property group the individual sheet falls into without reliance on extrapolation from tests on a small sample from an averall population. Furthermore, since the sheets are tested in a number of different positions over the length of a sheet, the test result for the whole sheet is far more accurate since any localised defects in the sheet would be more likely to be detected.

According to an aspect of the present invention there is provided; an appartus for the continuous testing of planar panels of particulate or layered material fed through the apparatus from an entry station to an exit station; said apparatus comprising a support structure having an array of rollers configured to facilitate the continuous feeding and sandwiching of said panels therebetween, means applying a load to the panels to enable the measurement of the structural properties of the continuously fed panels so as to include a deflection in the panels to facilitate said measurement.

In the preferred embodiment the load is applied by a loading roller which induces a deflection in the panel at a point on the panel equidistant from feed and exit rollers which are included in the roller configuration.

a method for the continuous testing of particulate board, utilising the apparatus as hereinbefore described, comprising the steps of;

(a) feeding particulate panel board through an array of rollers mounted on the support structure of the apparatus;

(b) locating the particulate board so that its leading edge is free of sandwiching exit rollers with a trailing portion of the particulate board held between sandwiching infeed rollers,

(c) inducing bending in the particulate panel board by means of the load roller which sits radially proud of the common plane of radial extremities of guide rollers thereby inducing deflection of the board,

(d) measuring structural parameters of the particulate panel board via a load cell linked to the load roller,

(e) repeating the measurements at predetermined intervals as the particulate board advances from the sandwiching infeed to outfeed rollers.

In another broad form according to the method aspect of the invention, the present invention comprises; a method for testing continuously the stiffness and other properties of a particulate of layered sheet material comprising the steps of:

(a) feeding the material continuously through a configuration of rollers;

(b) feeding the material to a distance such that part of the material is held by infeed rollers and leading part of the material is held by exit rollers;

(c) applying a force via a load roller to induce bending in the material at a point substantially central to the infeed and exit rollers;

(d) conducting repeated measurements at predetermined intervals as the sheet material passes continuously therethrough;

(e) calculating the stiffness factor of the panel.

In an alternative form the invention comprises:

an apparatus for the continuous testing of planar panels of particulate or layered material fed through the apparatus from an entry station to an exit station; said apparatus comprising a support structure having a array of rollers configured to facilitate the continuous feeding and sandwiching of said panels therebetween, means applying a load to the panels to enable the measurement of the structural properties of the continuously fed panels by inducing a deflection in the panels to facilitate said measurement.

According to the preferred embodiment the entry and exit rollers have associated therewith but spaced therefrom additional panel supporting rollers.

The present invention will now be described in more detail according to a preferred but non limiting embodiment and with reference to the accompanying illustrations wherein:

Figure 1: shows a isometric view of a preferred embodiment of the invention.

Figure 2: shows a schematic side elevational view of the apparatus according to a preferred embodiment of the present invention with the load roller disposed underneath the particulate panel board.

Figure 3: shows an alternative configuration of the apparatus of the present invention this time with the load roller disposed on top of the particulate panel board.

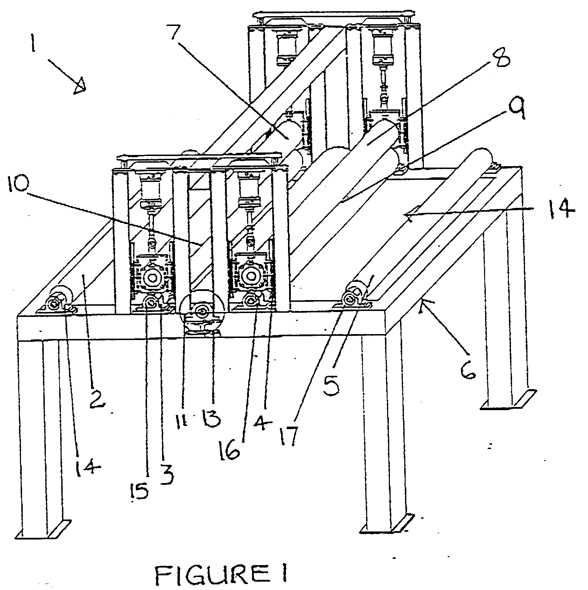

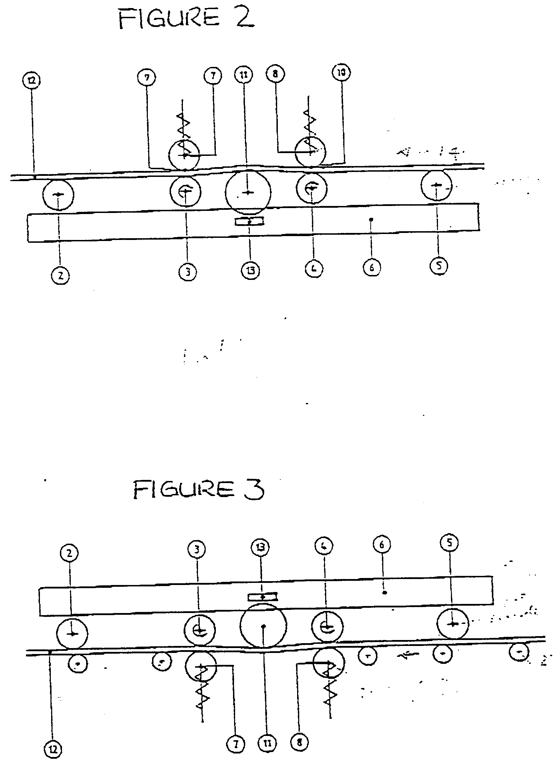

Referring to figure 1 there is shown an apparatus 1 comprising an assembly of rollers for receiving and supporting a continuously fed panel 12 (see figures 2 and 3) of particulate of layered material. The assembly of rolers includes fixed mounted rollers 2, 3, 4 and 5, which are fixed via their respective bearing housings 12, 14, 16 and 17 onto a rigid support structure 6.

Rollers 2, 3, 4 and 5 are in horizontal alignment with any one or all of these rollers acting together or individually as a driven roller.

According to the preferred embodiment superimposed above rollers 3 and 4 are displaceably biased sandwiching rollers 7 and 8 which apply sufficient pressure in a direction opposing rollers 3 and 4 so that an entry nip 9 and an exit nip 10 are formed respectively.

The spring biased rollers 7 and 8 are actuated by biasing mechanisms 18 and 19 at either end of these rollers provide one means for applying the requisite load to form the nip however, it is conceivable that the same effect can be achieved by utilisation of pneumatic of hydraulic cylinders or torque arms.

A further roller, load roller 11 is preferably located intermediate nips 9 and 10 and mounted on load cells which measure the deflection load applied onto the roller 11 generated in the panel 12 when it is fed into the apparatus 1. Roller 11 is located so that its radial distance protrudes beyond the upper plane of alignment of rollers 2, 3, 4 and 5. When a panel is to be measured for stiffness and other structural parameters which derive therefrom, the panel is sandwiched between rollers 3 and 7 and 4 and 8. Rollers 3 and 7 and 4 and 8 exert sufficient biasing pressure on the panel 12 to ensure contact with roller 11 so that a deflection upwards in the panel is created at roller 11. Load cell 13 in conjunction with roller 11 measures the deflection load thus enabling the stiffness and related parameters of the panel to be calculated at any point along the panel.

As the panel moves through the apparatus in the direction of arrow 14, measurements of stiffness can be taken at any point and at any time as the panel moves through the apparatus 1. The testing conditions are uniform for the period of time in which the panel 12 spans over the rollers 2, 3, 4 and 5 simultaneously.

The loading effect created by the assembly and in particular load roller 11 is constant whilst ever the panel spans over 3 spans. Those spans are respectively rollers 4 and 5, 3 and 4 and 2 and 3. As soon as the panel 12 leaves roller 2 due to the cantilevering effect created on the panel, a different result would be achieved at the deflection point above roller 11. Rollers 2, 3, 4 and 5 (see fig 2) therefore are needed for support of the board to guarantee uniform testing conditions and to eliminate any adverse bending effects from cantilevering of the panel at either end.

The non existence of the rollers 2 and 5 would allow the weight of the board which protudes past roller 3 on the outfed side or roller 4 on the infeed side to alter the deflection force occurring at roller 11. Since the board is moving through the apparatus, the degree of overhand thus, cantilevered board weight and resultant force on roller 11 would continually vary. This effect is therefore eliminated by rollers 2 and 5 as there is no longer an unwanted bending moment created by catilevering of the board to affect the deflection behaviour of the board over the load roller.

The load cell readings feed into a suitable micro processor, PLC of PC for processing for the desired output. This equipment can be used to display the load readings in real time, store them to files, compute statistical analysis and grade and label the panels according to the respective properties found from testing.

It will be obvious that the front and rear part of the panel 12 is not tested as the board feeds into the machines or tails out as the loading and deflection conditions are not uniform or constant as the board does not span over all of the rollers 2, 3, 4 and 5.

Since relatively short spans particularly between rollers 3 and 4 would result in high bending moment of the sheet, there is a limitation on how short the span may be between the sandwiching rollers 3, 7, 4 and 8. Generally, the spans as well as the board deflection will depend upon the thickness and stiffness range of the boards to be tested.

In the assembly described schematically in figure 2, the load cells not only sense the force required to deflect the board but also the weight the board that rests on the load roller 11 at any given time. Since this weight force is little compared to the deflection force, it generally can be neglected. If it needs to be considered it can be allowed for in the calibration (zero setting) of the load cells assuming that the board weight only varies slightly.

In an alternative embodiment as shown in figure 3, the apparatus 1 can be reconfigured so that the loading roller 11 applies a downward deflection to the panel 12. Figure 3 is essentially an inversion of the configuration shown in figure 1. In this embodiment the only minor difference would be that rollers 2 and 5 would not assist in the transport into the apparatus and that a suitable arrangement for that purpose would have to be found.

In the case of thickness variations of the board to be measured, a correct EI value could be determined but not a modulus of elasticity. For this purpose it would be possible to measure the board thickness on line as the board is tested and incorporate the actual thickness so measured into the elasticity calculations.

Since the stiffness of composite wood panels is correlated to the panels temperature as well as moisture content, varying temperature and moisture content of the panels to be measured will affect the result. If the correlations are known then the panel temperature and moisture content can also be measured on line and suitable calibrations or correction factors can be incorporated into the stiffness and elasticity calculations. These calculations would be conducted according to known and conventional mathematical methods. The present invention has numerous advantages over the prior art. Firstly, the process is continuous and non destructive. The panel deflection and deflection span is constant with the panel centrally deflected while spanning over three fixed spans so that the testing centre is not affected by effects from positions on the panel remote from the testing centre. The thickness of the panel is measured on-line for incorporation in the elasticity calculation. The moisture content and temperature of each panel tested can be measured for the correction of the stiffness/elasticity results.

The calculated result is displayed in real time or from memory, in the form of a graph or figures on a monitor of screen, this methodology essentially employs known computer technology.

The end result of the testing enables the stress grade or property group in which the panel falls to be correctly ascertained. The results can be collated for statistical and quality control and/or process control purposes with the results being used to monitor panel manufacturing processing.

It will be recognised by persons skilled in the art that numerous variations and modifications can be made to the present invention as broadly described herein without departing from the overall spirit and scope of the invention.

## Claims

1. An apparatus for the continuous testing of planar panels of particulate or layered material fed through the apparatus from an entry station to an exit station; said apparatus comprising a support structure having an array of rollers configured to facilitate the continuous feeding and sandwiching of said panels therebetween; means applying a load to the panels to enable the measurement of the structural properties of the continuously fed panels by the inducement of the deflection in the panels resulting from said load to facilitate said measurement.

2. An apparatus according to claim 1 wherein said array of rollers comprises support and/or guide rollers beneath or above said continuous panel and at least one load applying roller above or below said panel and at least two biasing rollers opposing said at least one load applying roller.

3. An apparatus according to claim 2 wherein the said loading roller/s induce a predetermined load onto the panels to induce said deflection therein.

4. An apparatus according to claim 3 wherein two biasing rollers are mounted on the opposite side of the panel to the said at least one load roller with each biasing roller eccentrically opposing said at least one primary load roller.

5. An apparatus according to claim 4 wherein there is one primary load roller which is equidistant from and between each of said biasing rollers.

6. An apparatus according to claim 5 wherein the primary load roller extends radially beyond the plane at which each of said first row of support or guide rollers touch said panel at their radial extremities.

7. An apparatus according to claim 6 wherein the said load roller is mounted on load cells enabling measurement of the parameters affected by said deflection load applied by said load roller.

8. An apparatus according to claim 7 wherein the said biasing rollers are adapted with a mechanism at either end to facilitate displacement along a vertical plane in response to infeed of said panel.

9. An apparatus according to claim 8 wherein a row of rollers is located below the said panel to act as support rollers with said load roller being located intermediate two of said support rollers.

10. An apparatus according to claim 9 wherein the biasing rollers are above the said panel.

11. An apparatus according to claim 10 wherein the said biasing rollers are biased downwardly toward the panel to positively sandwich the panel between the biasing rollers and the guide rollers.

12. An apparatus according to claim 8 wherein a row of rollers is located above said panel which act as guide rollers and load roller also being disposed above said panel and intermediate two of said guide rollers.

13. An apparatus according to claim 12 wherein the said biasing rollers are disposed beneath the said panel.

14. An apparatus according to claim 13 wherein the said biasing rollers are biased upwardly to positively sandwhich the panel between said biasing rollers and said guide rollers.

15. An apparatus according to claim 14 further comprising a row of support rollers beneath said panel.

16. An apparatus according to claim 15 wherein the forces applied to the continuous panel are constant or variable.

17. An apparatus according to claim 16 wherein the load roller is fixed or adjustable.

18. An apparatus according to claim 17 wherein the panel deflection is constant.

19. An apparatus according to claim 18 wherein the spans between the support and and guide rollers are equal.

20. An apparatus according to claim 19 wherein the panel is centrally deflected while spanning over three fixed spans between guide and or support rollers.

21. An apparatus according to claim 20 wherein the support biasing and guide rollers are fixed apart from rotation.

22. An apparatus according to claim 21 wherein the said load cell is interfaced into an associated computer processor.

23. A method for the continuous testing of particulate board, utilising the apparatus as hereinbefore described, comprising the steps of;
    (a) feeding particulate panel board through an array of rollers mounted on the support structure of the apparatus;
    (b) locating the particulate board so that its leading edge is free of sandwiching exit rollers with a trailing portion of the particulate board held between sandwiching infeed rollers,
    (c) inducing bending in the particulate panel board by means of a load roller which sits radially proud of the common plane of radial extremities of guide rollers thereby inducing deflection of the board,
    (d) measuring structural parameters of the particulate panel board via a load cell linked to the load roller,
    (e) repeating the measurements at predetermined intervals as the particulate board advances from the sandwiching infeed to outfeed rollers.

24. A method according to claim 23 comprising the further step of calculating strength properties of the particle panel board from the said measured parameters.

25. A method according to claim 24 wherein the panel deflection is maintained constant.

26. A method according to claim 25 wherein the spans between the guide rollers are equal.

27. A method according to claim 26 wherein the panel is centrally deflected while spanning over three fixed spans.

28. A method according to claim 27 wherein the guide rollers are fixed apart from rotation.

29. A method according to claim 28 wherein the parameters measured include stiffness, shear, bending, compression strength and tensile strength on the face of the particulate board not adjacent the load roller.

30. A method according to claim 29 wherein the load cell is interfaced into a computer processor for calculating the results of the testing.

31. A method according to claim 30 comprising the further step of calibrating to allow for panel moisture, temperature and weight.

FIGURE 1

# FIGURE 2

# FIGURE 3